(19) Europäisches Patentamt
European Patent Office
Office européen des brevets

(11) **EP 3 381 929 A1**

(12) **EUROPEAN PATENT APPLICATION**

(43) Date of publication:
**03.10.2018 Bulletin 2018/40**

(51) Int Cl.:
*C07K 7/06* *(2006.01)*       *A61K 49/22* *(2006.01)*

(21) Application number: **17164304.2**

(22) Date of filing: **31.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Rigshospitalet**
**2100 Copenhagen (DK)**
• **The University of Copenhagen**
**1165 Copenhagen K (DK)**

(72) Inventors:
• **Hempel, Casper**
**2100 Copenhagen (DK)**
• **Nielsen, Lars Bo**
**2100 Copenhagen (DK)**
• **Sillesen, Henrik**
**2100 Copenhagen (DK)**

(74) Representative: **Aera A/S**
**Gammel Kongevej 60, 18th floor**
**1850 Frederiksberg C (DK)**

(54) **TARGETED MICROBUBBLES FOR DETECTION OF GLYCOCALYX**

(57)    The present invention relates to a method for *in vivo* detection of the loss and/or presence of endothelial glycocalyx by using labelled microbubbles and ultrasound. The microbubbles are labelled with peptides via a lipid recognising specific components of the glycocalyx.

Fig. 1

EP 3 381 929 A1

**Description**

FIELD OF THE INVENTION

The present invention relates to a method for *in vivo* detection of the loss and/or presence of endothelial glycocalyx by using labelled microbubbles and ultrasound. The microbubbles are labelled with peptides via a lipid recognising specific components of the glycocalyx.

BACKGROUND OF THE INVENTION

Targeted microbubbles are known in the art, WO11/139927 for example describe targeted microbubbles, which are generated by post-labelling buried ligand microbubbles. WO11/139927 disclose a ligand, cyclic arginine-glycine-asparagine (RGD) which, is attached to the distal end of the poly(ethylene glycol) (PEG) moieties on the surface of microbubbles. The concept is that buried-ligand architecture may significantly reduce ligand-mediated immunogenicity. WO11/139927 does not mention targeting the glycocalyx.

Lindner et all, shows that "Albumin Microbubble Persistence During Myocardial Contrast Echocardiography Is Associated With Microvascular Endothelial Glycocalyx Damage" - Circulation. 1998: 2187-2194. In this article, Lindner et all. shows that shows that sonicated albumin microbubbles persist within the myocardium in situations in which the endothelial glycocalyx is damaged. The measurement of the myocardial transit rate of albumin microbubbles may provide an *in vivo* assessment of glycocalyx damage. Lindner et all does not mention a direct binding of a microbubble with a labeled peptide to a component of the glycocalyx, but measures indirectly.

WO00/42988 disclose that persistence of albumin microbubbles may be due to a charge specific interaction between these anionic microbubbles and the vascular endothelium in regions where there is loss of the negatively-charged glycocalyx, which coats the luminal surface of the endothelium. WO00/42988 does not mention a direct binding of a microbubble with a labeled peptide to a component of the glycocalyx.

Imaging the glycocalyx has been technically demanding since no standard procedures have been developed. The current methods included electron and fluorescent microscopy, thus there is an unmet need for non-invasive quantification of the glycocalyx volume in the microcirculation making it a useful diagnostic measure for e.g. diabetes patients.

SUMMARY OF THE INVENTION

In its broadest aspect, the present invention relates to microbubbles comprising a targeting moiety, wherein said targeting moiety selectively binds to endothelial glycocalyx.

In another aspect, the present invention relates to methods for determining the level of endothelial glycocalyx comprising measuring the binding of a microbubble according to the invention using an ultrasound device.

A third aspect of the invention relates to diagnosing and/or monitoring diseases related to changes in the level of endothelial glycocalyx, such as but not limited to Arteriosclerosis, Diabetes, Sepsis and Malaria.

A forth aspect of the invention relates to kit of parts needed by the end-user to prepare the inventive microbubbles comprising a targeting moiety aimed at the endothelial glycocalyx, and instructions for the use in e.g. diagnosing disease severity like Arteriosclerosis, Diabetes, Sepsis and Malaria.

DETAILED DESCRIPTION OF THE INVENTION

The present inventors have in the Examples shown the binding of labelled or targeted SonoVue microbubbles to human endothelial cells *in vitro.* Thus, in one embodiment, the present invention relates to a microbubble comprising a targeting moiety, wherein said targeting moiety selectively binds to endothelial glycocalyx. Unlike indirect measurement of the endothelial glycocalyx through e.g. adherence of microbubbles to sites with glycocalyx damage, the present invention provides more accurate means for quantification of the glycocalyx volume. Such alternative method for quantification of the endothelial glycocalyx provides several advantages especially in prognosis, diagnosis and monitoring of various endothelial glycocalyx-related diseases.

A targeting moiety in the present context relates to a part of a molecule that binds to a target through a molecular binding. A molecular binding in the present context is an attractive interaction between two molecules that results in a stable association in which, the molecules are in close proximity to each other. This may for example be in form of a non-covalent, reversible covalent or irreversible covalent binding.

*Microbubbles*

The microbubbles are echo contrast agents. Several methods of preparation of "smart" microbubbles for ultrasound image-guided therapy are known in the art.

Targeted ultrasound contrast agents can for example be prepared by attaching targeting ligands to the lipid, protein or polymer shell coating of gas-filled microbubbles. These materials are stable on storage, fully biocompatible and can be administered parenterally. The gas improves echogenicity and thus aid in detection of the microbubbles.

Due to the gas detection of contrast enhanced microbubbles by ultrasound is very efficient. Single particles with picogram mass can be visualized. Covalent or noncovalent binding techniques can be used to attach targeting ligands. Ligand-carrying microbubbles adhere to the respective molecular targets *in vitro* and *in vivo.* Several biomechanical methods are available to improve targeting efficacy, such as the use of a flexible tether spacer arm between the ligand and the bubble, and the use of folds on the microbubble shell, that project out, enhancing the contact area and increasing the length of the lever arm.

*Concentration*

The concentration of the glycosaminoglycan targeted microbubbles is between 100 and 500 x $10^6$ per ml, such as between 200 and 500 x $10^6$ per ml, 300 and 500 x $10^6$ per ml, 400 and 500 x $10^6$ per ml, 100 and 400 x $10^6$ per ml, 200 and 400 x $10^6$ per ml, 300 and 400 x $10^6$ per ml, 300 and 500 x $10^6$ per ml, 300 and 400 x $10^6$ per ml, 100 and 200 x $10^6$ per ml, 200 and 300 x $10^6$ per ml, 300 and 400 x $10^6$ per ml, and 400 and 500 x $10^6$ per ml.

*Size*

Microbubbles are typically bubbles smaller than one millimetre in diameter, but larger than one micrometre. The microbubbles of the invention have a mean bubble diameter of 2.5 $\mu$m and more than 90% of the bubbles are smaller than 8 $\mu$m. The size of the individual microbubbles do not permit passage through the endovascular borders and therefore remain as truly intravascular indicators.

*Safety*

Both patient deaths and other serious cardiopulmonary reactions have been reported related with, but not directly caused by microbubbles. Thus, there is a 'black box warning', which indicated that serious cardiopulmonary reactions, including fatalities, occurred during or within 30 min following the administration of microbubbles. These are rare cases though and the microbubbles of the present invention are safe, efficacious, and highly valuable diagnostic and therapeutic options for improving the health and well-being of patients.

All commercially available microbubbles mentioned below have received product labelling and safety indications by e.g. the FDA. The safety profile is generally very good.

The Sonovue microbubbles used in the Examples is frequently used in the EU due to very low number of side effects. For example, Optison has been reported to have a higher incidence of anaphylactic shock compared with Sonovue. Thus, Sonovue microbubbles may have a higher safety.

*Commercialized microbubbles*

Several commercialized microbubbles are on the market, such as Optison (1997), Definity (2001), Sonovue (2001), Luminty (2006), Sonazoid (2007), and Lumason (2014). Thus, the microbubbles of the present invention may be selected from the group consisting of commercialized microbubbles such as Optison, Definity, Sonovue, Luminty, Sonazoid, and Lumason. Over the years, multiple other agents were developed, but many are no longer in production. However, there are of course some important differences amongst the commercialized microbubbles.

The examples shown in the present application are generated by using Sonovue.

*Optison*

Optison microbubbles consist of octalfluoropropane gas encapsulated by a serum albumin shell. Produced by GE Healthcare (Princeton, NJ, USA). This product is only rarely used in Europe due to an uncertain frequency of patients developing allergic reactions (anaphylactic shock), yet the microbubbles of the present invention may be Optison based.

In one embodiment, the microbubbles are not Optison based.

*Definity and Luminty*

Definity and Luminty also consist of microspheres with an outer lipid shell that encapsulates perflutren. Produced by Lantheus Medical Imaging (Billerica, MA, USA).

Definity has been linked to a very low incidence of non-fatal events that are termed complement activation-related pseudo allergy, and these events may range from temporary back pain to hypotension to hypoxemia to angioedema.

However, the microbubbles of the present invention may be Definity and/or Luminty based.

In one embodiment, the microbubbles of the present invention are not Definity based.

In one embodiment, the microbubbles of the present invention are not Luminty based.

*Sonazoid*

Sonazoid consists of microspheres with an outer lipid shell that encapsulates perfluorobutane gas. It is produced by GE Healthcare and is marketed by Daiichi Pharmaceutical Co., Ltd (Tokyo, Japan).

Sonazoid is contraindicated in patients with known egg allergies and has been associated with a low incidence of side effects, such as diarrhea, albuminuria, and neutropenia. Thus, the microbubbles of the present invention may be Sonazoid based.

In one embodiment, the microbubbles of the present invention are not Sonazoid based.

*SonoVue and Lumason*

Sonovue and Lumason consist of microspheres with an outer lipid shell that encapsulates sulfur hexafluoride gas. They are produced by Bracco Imaging S.p.A (Milan, Italy). Thus, the microbubbles of the present invention may SonoVue and/or Lumason based.

Sonovue is contraindicated for those patients with hypersensitivity to the active substances, patients known to have right-to-left shunts, severe pulmonary hypertension (pulmonary artery pressure >90 mmHg), or uncontrolled systemic hypertension, and patients with acute respiratory distress syndrome.

SonoVue is made of microbubbles stabilized by phospholipids and containing sulphur hexafluoride. The suspension of the microbubbles is stable over the time following reconstitution.

The microbubbles of the invention can be sulphur hexafluoride microbubbles. The shell of the microbubbles of the invention can be made from lipid. The lipid may be a phospholipid.

In one embodiment, the shell of the microbubbles of the invention is not made from a protein.

In one embodiment, the shell of the microbubbles of the invention is not made from a globular protein.

One advantage of SonoVue microbubbles is the risk of anaphylactic shock is less since it is not based on human albumin. Another advantage is that SonoVue microbubbles are liposomes in which the lipid composition can be modulated enabling specific targeting.

The targeting ligand used for Sonovue may work for other types of microbubbles. Optison represents the least similar since it is based on a protein shell (albumin). Using the described method can also make other microbubbles including Optison bind specifically to the glycocalyx.

*Peptide*

The endothelial glycocalyx interacts with multiple proteins in the circulation. These proteins have a glycan-binding domain that have an overall positive charge, typically in clusters and sometimes within the so-called Cardin-Weintraub motif. In particular, binding to heparan sulfate has been studied in depth and a wide range of proteins have heparin-binding domains, which also interacts with heparan sulfate. Since the glycosaminoglycans are negatively charged it has been studied whether specific amino acids are involved in binding glycosaminoglycans. The peptides of the invention all disclose specific binding to the glycocalyx, and were initially designed having the general structure R(n)C (n = 3-9, where cysteine (C) was added for peptide conjugation) were synthesized and their interaction with heparin was determined by isothermal titration calorimetry.

Thus, the targeting moiety of the invention may comprise a functionalized lipid coupled to a peptide that enable specific binding to a glycosaminoglycan of the endothelial glycocalyx.

In a presently preferred exemplary embodiment of the invention, the peptide binds to a glycosaminoglycan of the endothelial glycocalyx.

*Positively charged peptides*

Glycosaminoglycan of the invention interacts most tightly with peptides (or peptide sequences within proteins) containing a complementary binding site of high positive charge density.

Thus, the peptides of the invention may have positive charge density.

Heparan sulfate, having fewer and more highly spaced negatively charged groups, interacts most tightly with a complementary site on a peptide (or peptide sequences with proteins) that has more widely spaced cationic residues.

In a presently preferred exemplary embodiment of the invention, the peptide capable of binding to a glycosaminoglycan is of this structure **C-RRRRG$_m$RG$_m$.**

The present inventors showed in the Examples a peptide with m=3. This structure is comparable to that found in Indian and desert hedgehog proteins.

In a presently preferred exemplary embodiment of the invention, the peptide capable of binding to a glycosaminoglycan is **CGRRRGGGRGG.**

Multiple proteins have a heparin-binding domain. The domains are tested *in vitro* and *in vivo* by conjugating them to microbubbles via for example cysteine.

Other peptides capable of binding to a glycosaminoglycan may be Indian hedgehog: **C-GSRRRPPRKL,** ApoE: **C-GERLRARM** or AAMP: **RRLRRMESESES-C.**

*Glycosaminoglycans*

Glycosaminoglycans (GAGs) or mucopolysaccharides in the present context are long unbranched polysaccharides consisting of a repeating disaccharide unit.

Members of the glycosaminoglycan family vary in the type of hexosamine, hexose or hexuronic acid unit they contain (e.g. glucuronic acid, iduronic acid, galactose, galactosamine, glucosamine). They also vary in the geometry of the glycosidic linkage. Examples of GAGs include Chondroitin sulfate, Dermatan sulfate, Keratan sulfate, Heparin, Heparan sulfate, and Hyaluronan.

*Heparan sulfate*

In one exemplary embodiment of the invention, the glycosaminoglycan is Heparan sulfate. Heparan sulfate is the most abundant glycosaminoglycan on the endothelial surface. The long disaccharide repeats are structured in domains with etiher low or high degree of sulfation. It is predicted that particularly the areas with a high degree of sulfation will enable binding of microbubbles with a positively charged peptide.

*Chondroitin sulfate*

In another exemplary embodiment of the invention, then glycosaminoglycan is Chondroitin sulfate. Chondroitin sulfate is less abundant than heparan sulfate, but is also expressed in high levels on the endothelial surface. There are multiple types of sulfation of chondroitin sulfate which may result in rather specific binding of the microbubbles.

*Peptide Safety*

The peptides used in the present invention are either designed to bind negatively charged glycosaminoglycans or stem from the amino acid sequence from human proteins. In the former case, there is a risk that the immune system will generate antibodies against the microbubbles. There is little or no risk for this in the latter case when the peptides used are derived from human proteins. The sequence of the peptides in itself is not associated with either stress induced responses or programmed cell death (apoptosis). The peptides will be derived from proteins that normally bind to these glycosaminoglycan chains and thus the cell will likely only sense that something has bound as usually.

*Lipid coupling*

The present inventors use functionalised, PEGylated lipids that anchor themselves into the microbubbles. The lipid used in Example section is DSPE (1,2-distearoyl-**sn-**glycero-3-phosphoethanolamine), which is a saturated phospholipid. The PEG chain is covalently attached to the phospholipid and allows for long circulation times.

*Maleimide*

Two formats for conjugating peptides to bubbles were used in the Examples. Both methods involve maleimide coupling to the thiol group on a cysteine.

Thus, the lipid DSPE-PEG linker may for example be 880126 - DSPE-PEG(2000) Maleimide (Avanti Polar Lipids INC).

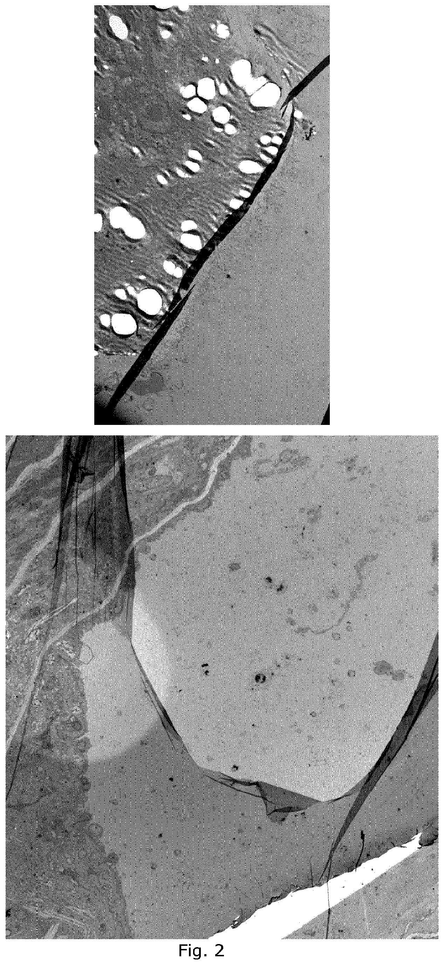

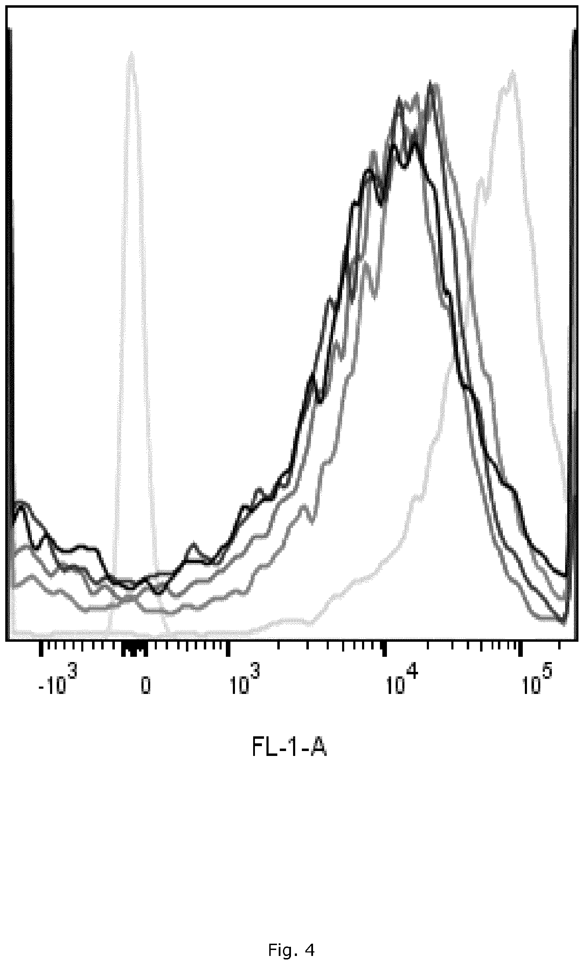

A maleimide is a chemical group that has strong and specific affinity for binding thiols. Initially, the loading of lipids and peptides to the microbubbles was tested. Lipids were initially of the type DSPE-biotin enabling a reaction with an avidin-conjugated fluorophore. Dose-dependent labelling was tested in a flow cytometer (fig. 4). Since stability of bubbles may be an issue the present inventors tested how bovine serum albumin affected stability. By incubating in albumin, the present inventors noticed a dose-dependent disruption of the particles within 10 minutes as seen in the patients (fig. 2).

The present inventors have optimized on how much peptide is conjugated to the bubbles. By using a FITC-conjugated lipid anchor as well as an anchor designed for peptide coupling binding to endothelial cells was assessed by microscopy.

The first coupling strategy employed was lipid-PEG-biotin + neutravidin-maleimide + peptide. The second strategy was lipid-PEG-maleimide + peptide. Both strategies have been successful. A fluorescently conjugated DSPE lipid may not be part of the clinical product but is only used for *in vitro* characterisation. This will influence binding to cells.

*Biotin*

Biotin is widely used throughout the biotechnology industry to conjugate proteins for biochemical assays - biotinylation. Biotin's small size means the biological activity of the protein will most likely be unaffected. Biotin has a very strong binding affinity to avidin (including neutravidin as employed in the Examples). Thus, the lipid DSPE-PEG linker may for example be 880129 - DSPE-PEG(2000) Biotin (Avanti Polar Lipids INC).

*Ultrasound*

Ultrasound according to the invention relates to medical ultrasound also known as diagnostic sonography or ultrasonography, and is a diagnostic medical procedure that uses sound waves to produce images on a screen, which allows medical providers to view internal structures of the body.

Typical sonographic instruments operate in the frequency range of 1 to 18 megahertz, though frequencies up to 50-100 megahertz have been used experimentally in a technique known as biomicroscopy in special regions, such as the anterior chamber of the eye.

The choice of frequency is a trade-off between spatial resolution of the image and imaging depth: lower frequencies produce less resolution but image deeper into the body. Higher frequency sound waves have a smaller wavelength and thus are capable of reflecting or scattering from smaller structures. Higher frequency sound waves also have a larger attenuation coefficient and thus are more readily absorbed in tissue, limiting the depth of penetration of the sound wave into the body. Sonography is effective for imaging soft tissues of the body. Superficial structures such as muscles, tendons, testes, breast, thyroid and parathyroid glands, and the neonatal brain are typically imaged at a higher frequency (7-18 MHz), which provides better axial and lateral resolution. Deeper structures such as liver and kidney are typically imaged at a lower frequency 1-6 MHz with lower axial and lateral resolution but greater penetration.

The sound waves of the present invention have frequencies which are higher than those audible to humans >20,000 Hz.

In one exemplary embodiment of the invention, the sound waves have frequencies between 5 and 12 MHz, rarely up to 15 mHz. In this range most analyses will be performed. Ultrasound is a harmless diagnostic tool and does not require anaesthesia or a large scanner. The transducer emits ultrasonic burst and detects its echo. The echo detected is then translated into anatomical structures since tissues echo differently.

However, since some of the echo is lost due to penetration through tissue the quality and precision of the detected echo/signal is best the closer to the skin is possible. Ultrasound will primarily be performed in the large arteries in the thigh and carotid artery since these large vessels lie close to the skin.

*Ultrasound device*

Ultrasonic waves are waves of frequency above the audible frequencies the human ear. In medical diagnostics are used ultrasound frequencies between 3 and 12 MHz.

The most important parameters describing the wave are (1):

Wavelength
Frequency
Velocity
Intensity

The first three characteristics are linked together by the formula:

$$v = fl$$

v-Velocity of ultrasound (approximately 1540 m/s in the soft tissues),
f-Frequency in Hz
l-Wavelength in m

In medical ultrasound diagnostics are used short pulses of ultrasound, which contain a whole range of frequencies. Human tissues are not homogeneous in terms of the ultrasonic waves, and the passage of waves through the tissue leads to refraction, reflection, scattering and absorption of energy.

Reflection depends on the characteristic acoustic impedance of the funds on whose border is reflected ultrasound. The absorption and refraction of ultrasound increases with frequency, i.e., lower frequencies are pervasive. Therefore, for abdominal examinations (liver, kidneys, pancreas) using a frequency of about 3 MHz, for examination of children, neck, breast, and the similar-around 5 MHz, and some times even 7 MHz. The higher frequency allows better discernment of detail in the picture, and is being used by the highest frequencies that are sufficiently pervasive.

*Glycocalyx measurement*

The present invention relates to methods for determining the level of glycocalyx. For example, the method may comprise

a) providing a microbubble capable of binding to a glycosaminoglycan

b) providing an ultrasound device configured to operate in the frequency range between 5 and 12 MHz

c) monitoring the binding of the glycosaminoglycan targeted microbubble to the glycocalyx with said ultrasound device

thereby determining the level of glycocalyx.

*In vivo measurements*

The methods of the present invention enable *in vivo* measurements, thus the effects of various levels of glycocalyx can be measured on whole, living organisms, including humans.

*In vitro measurements*

The methods of the present invention also enable *in vitro* measurements, thus the effects of various levels of glycocalyx can be measured on cells in a lab. One may wish to perform ultrasound imaging and one may wish to insert a fluorescent lipid as we have performed during the development of the product.

It should be understood that any feature and/or aspect discussed herein in connection with the determination or measurement of the glycocalyx according to the invention apply by analogy to the "diagnosis", "prognosis", "monitoring", "screening", "research purposes", "contact tracing", "enhanced case finding" and "prevalence studies" according to the invention and *visa versa*.

*Diagnosis*

Clinical utility of the glycocalyx may be assessed in comparison to and in combination with other diagnostic tools for the given disease. In short, the invention may relate to a method for determining the level of endothelial glycocalyx in a subject in need thereof, the method comprise measuring the binding of a glycosaminoglycan targeted microbubble to the endothelial glycocalyx using an ultrasound device configured to operate in the frequency range between 5 and 12 Hz.

In more detail, it is an object of preferred embodiments of the present invention to provide a method for diagnosing diseases having an alteration in the level of the glycocalyx, the method comprises

a) determining the level glycocalyx by for example

i) providing a microbubble capable of binding to a glycosaminoglycan
ii) providing an ultrasound device configured to operate in the frequency range between 5 and 12 MHz, and

iii) measure the binding of the glycosaminoglycan targeted microbubble to the glycocalyx with said ultrasound device

b) constructing a percentile plot of the glycocalyx level obtained from a healthy population

c) constructing a ROC (receiver operating characteristics) curve based on the glycocalyx level determined in the healthy population and on the glycocalyx level determined in a population who suffers from a glycocalyx related disease

d) selecting a desired specificity

e) determining from the ROC curve the sensitivity corresponding to the desired specificity

f) determining from the percentile plot the glycocalyx level corresponding to the determined sensitivity; and

g) predicting the individual to have a glycocalyx related disease, if the level of glycocalyx measured is equal to or lower than said glycocalyx level in the healthy population corresponding to the determined specificity and predicting the individual as unlikely or not to having a glycocalyx related disease, if the level of glycocalyx measured is higher than said total glycocalyx level in the healthy population corresponding to the determined specificity.

*Specificity and sensitivity*

The sensitivity of any given diagnostic test define the proportion of individuals with a positive response (i.e. the antigen response or the antigen-dependent response above the reference-level) who are correctly identified or diagnosed by the test, e.g. the sensitivity is 100%, if all individuals with a given condition have a positive test. The specificity of a given screening test reflects the proportion of individuals without the condition who are correctly identified or diagnosed by the test, e.g. 100 % specificity is, if all individuals without the condition have a negative test result. Sensitivity is defined as the proportion of individuals with a given condition, who are correctly identified by the described methods of the invention.

Specificity herein is defined as the proportion of individuals without the condition (e.g. disruption of the glycocalyx), who are correctly identified by the described methods of the invention (e.g. has decline in the glycocalyx level).

Receiver-operating characteristics Accuracy of a diagnostic test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease, latent or recent changes in the glycocalyx versus no change, or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results]. This has also been referred to as positivity in the presence of a disease or condition. It is calculated solely from the affected subgroup. On the x axis is the false-positive fraction, or 1 - specificity [defined as (number of false- positive results) / (number of true-negative + number of false-positive results)]. It is an index of specificity and is calculated entirely from the unaffected subgroup. Because the true-and false-positive fractions are calculated entirely separately, by using the test results from two different subgroups, the ROC plot is independent of the prevalence of disease in the sample. Each point on the ROC plot represents a sensitivity/- specificity pair corresponding to a particular decision threshold. A test with perfect discrimination (no overlap in the two distributions of results) has an ROC plot that passes through the upper left corner, where the true- positive fraction is 1.0, or 100% (perfect sensitivity), and the false- positive fraction is 0 (perfect specificity). The theoretical plot for a test with no discrimination (identical distributions of results for the two groups) is a 45[deg.] diagonal line from the lower left corner to the upper right corner. Most plots fall in between these two extremes. (If the ROC plot falls completely below the 45[deg.] diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always > 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

The specificity of the method according to the present invention may be from 70% to 100%, more preferably 80% to 100%, more preferably 90% to 100%. Thus in one embodiment of the present invention the specificity of the invention is 80%, 81%, 82%, 83%, 84%, 85%, 86%, 87%, 88%, 89%, 90%, 91%, 92%, 93%, 94%, 95%, 96%, 97%, 98%, 99% or 100%.

The sensitivity of the method according to the present invention may be from 70% to 100%, more preferably 80% to 100%, more preferably 90% to 100%. Thus in one embodiment of the present invention the sensitivity of the invention is 80%, 81%, 82%, 83%, 84%, 85%, 86%, 87%, 88%, 89%, 90%, 91%, 92%, 93%, 94%, 95%, 96%, 97%, 98%, 99% or 100%.

*Prognosis*

In one embodiment, determining the level of endothelial glycocalyx in a subject may be used for predicting the prognosis of subjects diagnosed with various vascular dysfunctions, such as but not limited to Atherosclerosis. When used in patient prognosis the method according to the present invention may help to predict the course and probable outcome of the endothelial glycocalyx states. Thus, assisting the skilled artisan in selecting the appropriate treatment method and predict the effect of a certain treatment for the condition.

*Monitoring*

In one embodiment, determining the level of endothelial glycocalyx in a subject may be used for monitoring subjects diagnosed with a disease related to endothelial glycocalyx. When used in patient monitoring the method according to the present invention may help to assess efficacy of treatment during and after termination of treatment e.g. monitoring and predicting possible recurrence of the endothelial glycocalyx.

*Glycocalyx in vascular endothelial tissue*

The glycocalyx is a carbohydrate-rich matrix layer that surrounds the cell membranes of endothelia cells. The glycocalyx plays a major role in regulation of endothelial vascular tissue, including the modulation of red blood cell volume in capillaries. The glycocalyx is located on the apical surface of vascular endothelial cells which line the lumen.

The glycocalyx is composed by glycoproteins and proteoglycans, the latter being the most dominant due to several long glycosaminoglycan chains. The glycocalyx may be lost upon a wide variety of extrinsic stimuli including inflammation, high glucose, cholesterol and oxidative stress. Disruption to this structure has detrimental effects that can cause disease. One of the functions of glycocalyx is that it works like a molecular sieve. Thus, upon loss endothelial permeability is increased leading to edemas in tissues.

*Vascular dysfunction*

In vascular diseases, endothelial dysfunction is a systemic pathological state of the endothelium (the inner lining of blood vessels) and can be broadly defined as an imbalance between vasodilating and vasoconstricting substances produced by (or acting on) the endothelium. The glycocalyx is a mechanosensor and has been associated with nitric oxide release (vasodilation). Consequently, when lost the vessel responsiveness is impaired.

*Atherosclerosis*

It has been shown that in macrocirculation the glycocalyx is significantly thinner in atheroprone areas compared with atheroprotected areas.

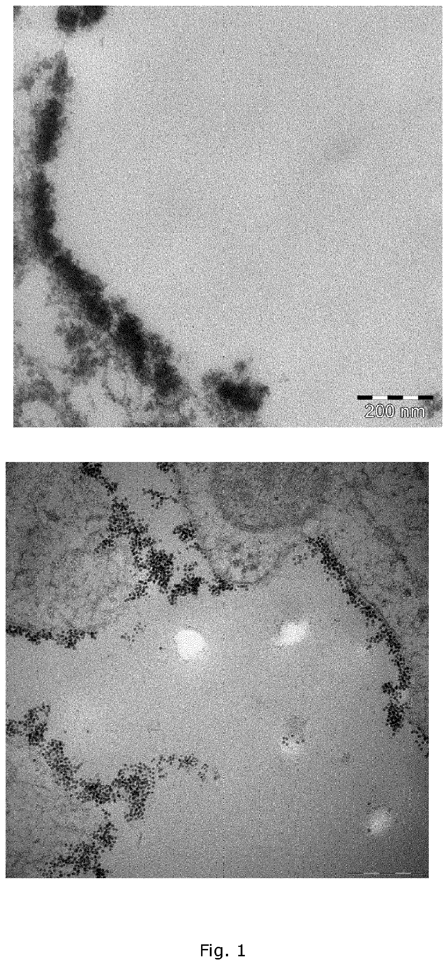

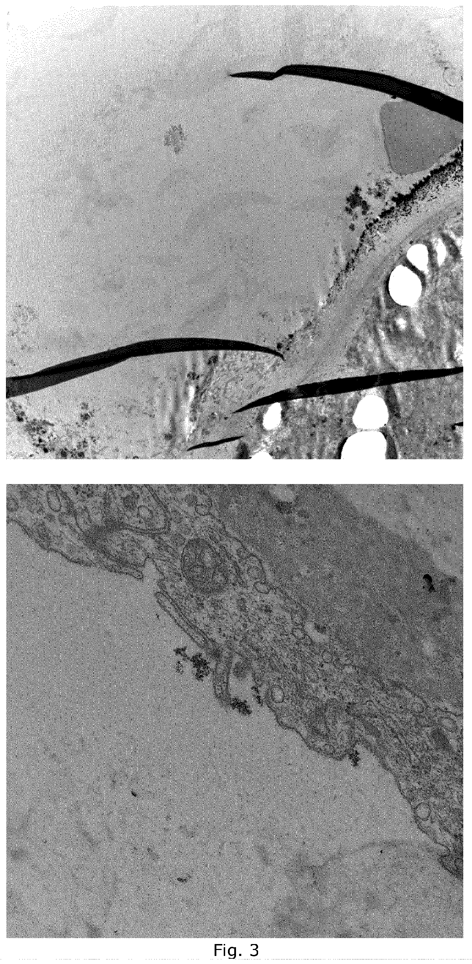

The present inventors show that loss of endothelial glycocalyx can be an initial step in atherosclerotic plaque formation and leads to accumulation of low-density lipoprotein-cholesterol (figure 1-3).

In one embodiment, the present invention can visualise the endothelial glycocalyx in the carotid artery, an artery with frequent presence of atherosclerotic plaque formation.

In one embodiment, the present invention can predict which plaques that will rupture and which would not based on the measured level of glycocalyx.

*Sepsis*

In sepsis, there is systemic inflammation. One of the consequences is increased activity of multiple enzymes. One of them is an endothelial glucosidase that cleaves off heparan sulfate from the endothelium. Reducing the level of glycocalyx loss have been associated with improved survival in experimental sepsis. Thus, obviously means for monitoring the level of glycocalyx may be important in patients suspected of having sepsis, and the present invention enables real time monitoring in a fast and reliable manner.

*Malaria*

Malaria is also a vasculopathy and has a strong inflammatory component. In malaria it has been shown that glycocalyx loss in the brain is dependent on severity of malaria. In the plasma, components of the glycocalyx are also seen in increased amounts. Thus, obviously means for monitoring the level of glycocalyx may be important in patients suspected of having malaris, and the present invention enables real time monitoring in a fast and reliable manner.

*Diabetes*

Patients with diabetes invariably show an impairment of endothelium-dependent vasodilation. Therefore, understanding and treating endothelial dysfunction is a major focus in the prevention of vascular complications associated with all forms of diabetes.
In diabetes mellitus the endothelial glycocalyx is compromised. It has been shown to be more permeable and significantly thinner both in patients and in experimental studies. Thus, obviously means for monitoring the level of glycocalyx may be important in patients suspected of having diabetes, and the present invention enables real time monitoring in a fast and reliable manner.

*Kit of parts*

The kit contains of one tube with freeze dried microbubbles. This tube also contains the specific peptide-conjugated lipids that bind the glycocalyx. This tube is sterile. The freeze-dried powder is solubilized in sterile isotonic saline and ready for iv-injection. Immediately, the contrast can be seen as enhanced and bubbles binding the glycocalyx will be noticed during ultrasound diagnosis.
In one embodiment, the invention relates to a kit of parts comprising

a) an untargeted microbubble

b) a functionalized lipid

c) a peptide that enable specific binding to the endothelial glycocalyx, and

d) instructions for preparing a targeted microbubble according to the invention.

In a further embodiment, the kit may further comprise instructions and means for use of a targeted microbubble according to the invention for the detection of endothelial glycocalyx.

*General*

It should be understood that any feature and/or aspect discussed above in connections with the microbubbles according to the invention apply by analogy to the methods described herein.
The following figures and examples are provided below to illustrate the present invention. They are intended to be illustrative and are not to be construed as limiting in any way.

BRIEF DESCRIPTION OF THE FIGURES

*Figure 1-3*
Transmission electron microscopy of glycocalyx in vessels. Healthy blood vessels are covered in a negatively charged glycocalyx. This can be stained with positively charged dyes. Figure 1 shows glycocalyx staining in healthy blood vessels. Figure 2 and 3 show loss of glycocalyx in areas of plaques. The column to the left is stained with lanthanides and the column to the right is stained with ferritin. Only few spots of negative charged can be detected with the dyes.

*Figure 4*

Microbubbles (Sonovue) labelled with DSPE-biotin at different concentrations and then detected with streptavidin-FITC. The histograms show intensity on the x axis and number of cells on the y axis. As can be seen there is a small concentration dependency but generally labelling is very efficient. Negative control is in pale blue and the highest concentration in green.

| Parameter | SE Dymax % Positive | Overton %Positive | Chi-Squared T (x) | K-S max Diff | K-S Max at Intensity | K-S Probability |
|---|---|---|---|---|---|---|
| Time | 4.3069 | 0.3140 | 0.0000 | 1.03 | 8.0002 | <80% |
| FSC-A | 5.9948 | 3.8440 | 4.2185 | 3.84 | 187.1231 | > 99.9% |
| SSC-A | 20.6883 | 7.4120 | 123.1088 | 9.80 | 662.9876 | > 99.9% |
| FL-1-A | 57.7772 | 50.4660 | 978.7344 | 50.5 | 24333.6579 | > 99.9% |

| | Sample Name | Count |
|---|---|---|
| COLOR - Black | Specimen_002_F1_F01.fcs | N/A |
| COLOR - Blue | Specimen_001_A1_A01.fcs | 10000 |
| COLOR - Pink | Specimen_002_E1_E01.fcs | 10000 |
| COLOR - red | Specimen_002_D1_d01.fcs | 10000 |
| COLOR – light blue | Specimen_002_C1_C01.fcs | 10000 |
| COLOR - green | Specimen_002_B1_B01.fcs | 10000 |

*Figure 5-7*

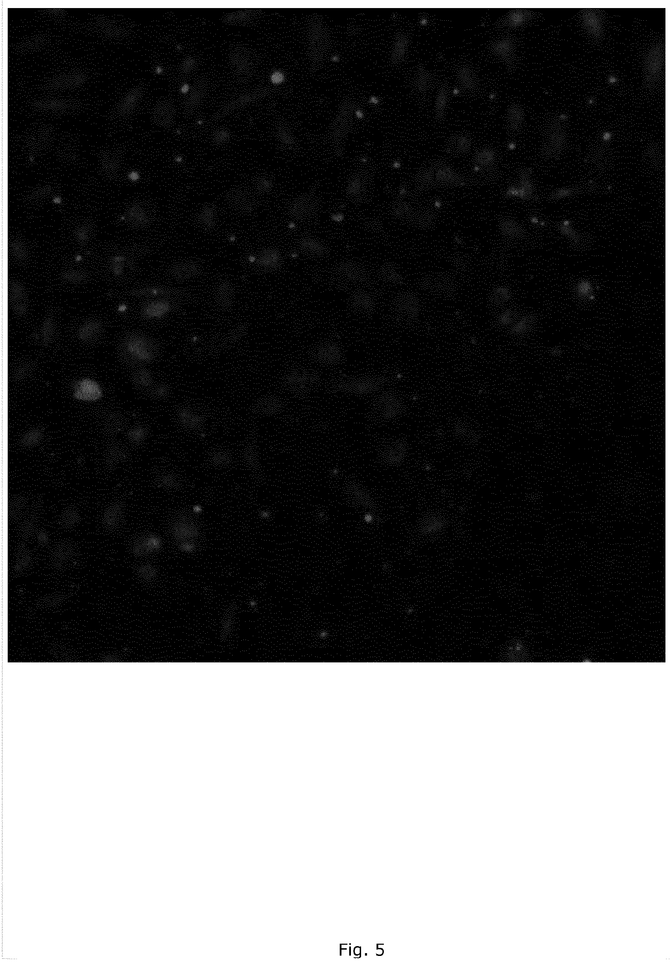

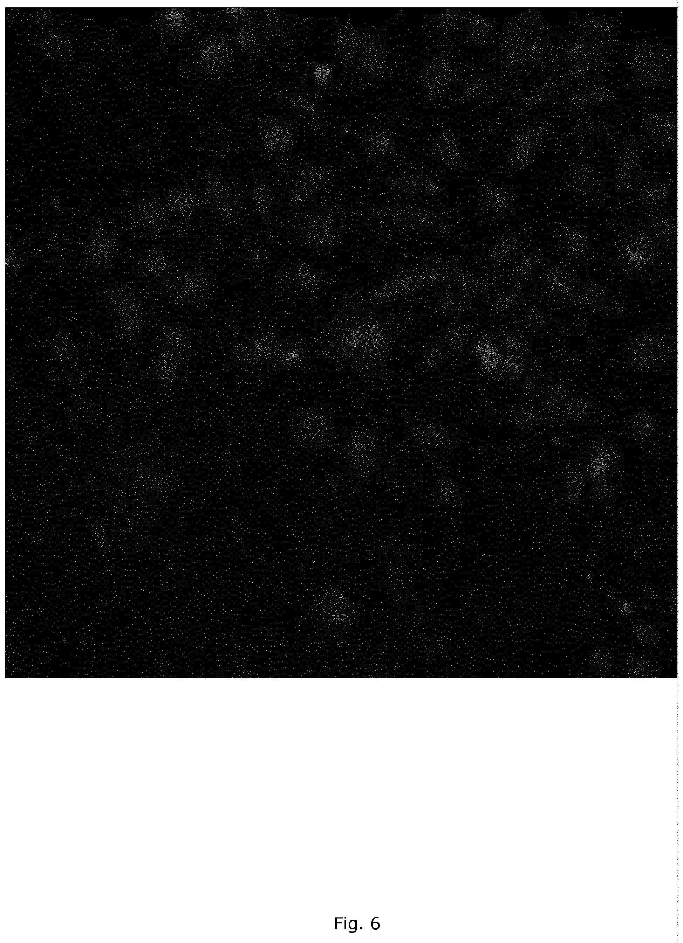

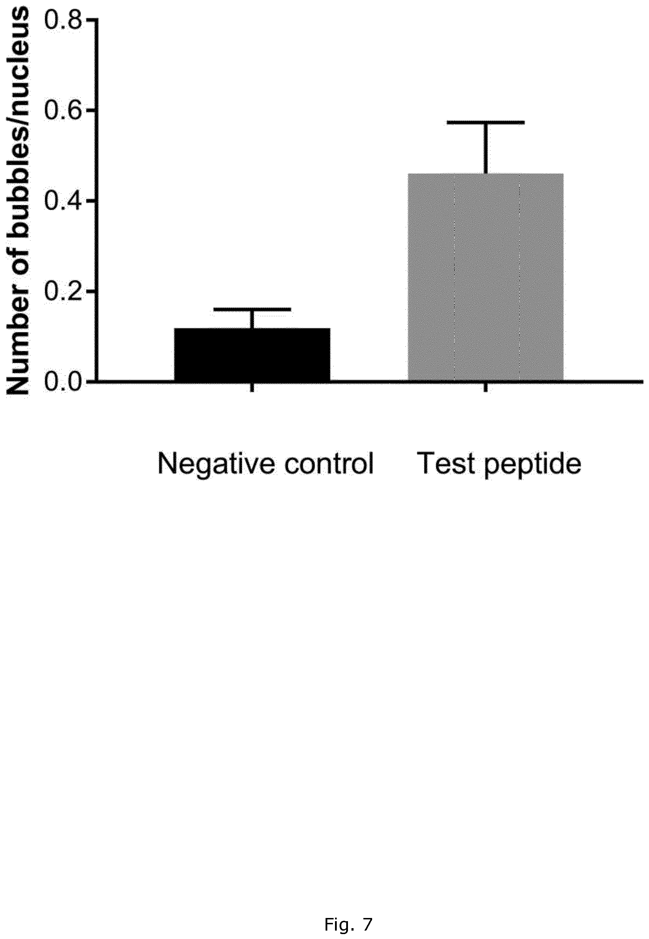

Binding of microbubbles to the endothelial cells is significantly improved when target peptides are present. Representative snapshots are seen: Scrambled peptide (fig. 6), target peptide (fig. 5) and quantification of the difference in binding in vitro (fig. 7). Microbubbles are green and nuclei are represented in red.

EXAMPLES

*EXAMPLE 1 - APO-E KO mice develop macroscopic plaques in the arteries as they age.*

To assess whether glycocalyx was present on the endothelial cells near the plaques mice were perfused with either cationized ferritin or lanthanum. Both these substances will bind the negatively charged glycocalyx. When analyzing the vessels with transmission electron microscopy we noticed only sporadic binding of these substances and in noticeable lower amount than in healthy vessels.

*EXAMPLE 2 - Microbubbles - (sonovue)*

The first attempt was aiming at targeting the microbubbles. We noticed that adding a DSPE-biotin lipid got embedded into the microbubble. This was assessed by staining the bubbles with streptatividin-FITC. If embedded, this will result in high fluorescence (FITC).

*EXAMPLE 3 - Peptides*

We tested binding of two different peptides to human primary aortic endothelial cells grown statically in vitro. The sequences of the peptides are:

Test peptide: **CGRRRGGGRGG**
Scrambled peptide: **CGSSSGGGSGG**

The main difference is the high number of positively charged peptide. After incubation with cells the number of bubbles were counted by using a fluorescent microscope. The bubbles had also DSPE-PEG-FITC incorporated. Nuclei were stained and binding was quantified as bubbles per nuclei in multiple wells and in multiple fields of view.

More peptides binds to the glycocalyx when anchored in microbubbles.

*EXAMPLE 4 - Lipid*

The lipid inserted into the microbubbles is DSPE. DSPE is conjugated to a PEG chain (2k), which is coupled either to biotin or maleimide. If the group is a biotion, neutravidin-actvated maleimide was used for binding to the biotin group. Maleimide was the most distal group and was thus able to bind cysteine on the peptide via its thiol group.
For characterization and quantification of binding, the lipids are also of the type DSPE-PEG(2k)-FITC.

*EXAMPLE 5 - In vivo assessment of ultrasound imaging in mice*

To assess that the microbubbles can bind to the glycocalyx in vivo mice is injected with bubbles plus/minus targeting peptides. If the microbubbles perform as in the in vitro settings, we can see them binding in large arteries. In this study APO-E KO mice with atherosclerotic plaques is included. From this we can assess the loss of glycocalyx in mice in vivo.

*EXAMPLE 6 -Vascular complications*

When seeing that the labelling strategy works in vitro and in vivo, method will also work in humans with vascular complications. These diseases include diabetes, atherosclerosis, sepsis and malaria. We can diagnose atherosclerosis and assess glycocalyx thickness in vivo. This will be useful data before a surgery. In diabetes it would be useful in terms of assessing how well vessels respond to diabetic therapy. In sepsis and malaria this measure would give indications on the degree of vascular complication and edema.

Example 7 - Specificity

Specificity for the glycosaminoglycan chains is addressed by pretreating endothelial cells in vitro by specific glycosidases. This way it can be assessed whether binding of peptides is dependent on heparan sulfate, chondroitin sulfate or both.

Example 8 - Toxicity

Acute toxicity is tested *in vitro.* This is made by standard assays and proteome profiling.

**Claims**

1. A microbubble comprising a targeting moiety, wherein said targeting moiety selectively binds to endothelial glycocalyx.

2. A microbubble according to claim 1, wherein the targeting moiety comprise a functionalized lipid coupled to a peptide that enable specific binding to a glycosaminoglycan of the endothelial glycocalyx.

3. A microbubble according to any of claims 1-2, wherein the peptide binds to a glycosaminoglycan of the endothelial glycocalyx.

4. A microbubble according to any of claims 1-3, wherein the glycosaminoglycan is Heparan sulphate or Chondroitin sulphate.

5. A microbubble according to any of claims 1-4, wherein the net charge of the peptide is positive.

6. A microbubble according to any of claims 1-5, wherein the peptide is CGRRRGGGRGG.

7. A microbubble according to any of claims 1-6, wherein the functionalized lipid is a DSPE-PEG linker.

8. A microbubble according to any of claims 1-7, wherein the functionalized lipid is a DSPE-PEG(2000) Maleimide.

9. A microbubble according to any of claims 1-8, wherein the functionalized lipid is a DSPE-PEG(2000) Biotin.

10. A microbubble according to any of claims 1-9, wherein the microbubble is a sulphur hexafluoride microbubble.

11. A method for determining the level of endothelial glycocalyx in a subject in need thereof, the method comprise measuring the binding of a microbubble according to any of claims 1-10, using an ultrasound device configured to operate in the frequency range between 5 and 12 Hz.

12. A kit of parts comprising

   a) an untargeted microbubble
   b) a functionalized lipid
   c) a peptide that enable specific binding to the endothelial glycocalyx, and
   d) instructions for preparing a targeted microbubble according to any of claims 1-10.

13. The kit according to claim 13, further comprising instructions and means for use of a targeted microbubble according to any of claims 1-10 for the detection of endothelial glycocalyx.

Fig. 1

Fig. 2

Fig. 3

FL-1-A

Fig. 4

Fig. 5

Fig. 6

Fig. 7

Europäisches
Patentamt

European
Patent Office

Office européen
des brevets

# EUROPEAN SEARCH REPORT

Application Number

EP 17 16 4304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOICHI NEGISHI ET AL: "AG73-modified Bubble liposomes for targeted ultrasound imaging of tumor neovasculature", BIOMATERIALS, vol. 34, no. 2, 1 January 2013 (2013-01-01), pages 501-507, XP055112949, ISSN: 0142-9612, DOI: 10.1016/j.biomaterials.2012.09.056 | 1-5,7,8, 11-13 | INV. C07K7/06 A61K49/22 |
| Y | * abstract; p. 502 * | 9,10 | |
| A | | 6 | |
| Y | WO 2004/006964 A1 (TARGESON LLC [US]; KLIBANOV ALEXANDER L [US]; LEY KLAUS F [US]; RYCHAK) 22 January 2004 (2004-01-22) | 9,10 | |
| A | * p. 12, 23 * | 1-8, 11-13 | |
| A | XIAOPING LENG ET AL: "Ultrasound Detection of Myocardial Ischemic Memory Using an E-Selectin Targeting Peptide Amenable to Human Application", MOLECULAR IMAGING, vol. 13, no. 4, 1 June 2014 (2014-06-01), page 1, XP055403702, ISSN: 1536-0121, DOI: 10.2310/7290.2014.00006 * abstract; p.2 * | 1-13 | |

TECHNICAL FIELDS SEARCHED (IPC)

C07K
A61K

The present search report has been drawn up for all claims

| Place of search | Date of completion of the search | Examiner |
|---|---|---|
| Munich | 5 September 2017 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 4304

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2017

| Patent document cited in search report | | Publication date | Patent family member(s) | | Publication date |
|---|---|---|---|---|---|
| WO 2004006964 | A1 | 22-01-2004 | AU | 2003251858 A1 | 02-02-2004 |
| | | | CA | 2532324 A1 | 22-01-2004 |
| | | | EP | 1551459 A1 | 13-07-2005 |
| | | | JP | 2006510579 A | 30-03-2006 |
| | | | US | 2005260189 A1 | 24-11-2005 |
| | | | WO | 2004006964 A1 | 22-01-2004 |

EPO FORM P0459

For more details about this annex : see Official Journal of the European Patent Office, No. 12/82

**EP 3 381 929 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 11139927 A
- WO 0042988 A

### Non-patent literature cited in the description

- **LINDNER.** Albumin Microbubble Persistence During Myocardial Contrast Echocardiography Is Associated With Microvascular Endothelial Glycocalyx Damage. *Circulation,* 1998, 2187-2194
- **ZWEIG, M. H. ; CAMPBELL, G.** *Clin. Chem.,* 1993, vol. 39, 561-577

SEQUENCE LISTING

<110> RIGSHOSPITALET COPENHAGEN UNIVERSTIY HOSPITAL
Hempel, Casper
Sillesen, Henrik
Nielsen, Lars Bo

<120> TARGETED MICROBUBBLES FOR DETECTION OF GLYCOCALYX

<130> P1299EP00

<160> 6

<170> PatentIn version 3.5

<210> 1
<211> 9
<212> PRT
<213> Artificial

<220>
<223> Heparan sulfate complementary motif

<220>
<221> misc_feature
<222> (2)..(2)
<223> Xaa can be any naturally occurring amino acid

<400> 1

Cys Xaa Arg Arg Arg Arg Gly Arg Gly
1               5

<210> 2
<211> 11
<212> PRT
<213> Artificial

<220>
<223> Heparan sulfate complementary peptide

<400> 2

Cys Gly Arg Arg Arg Gly Gly Gly Arg Gly Gly
1               5                   10

<210> 3
<211> 12
<212> PRT
<213> Artificial

<220>
<223> Heparan sulfate complementary Indian hedgehog

<220>
<221> misc_feature
<222> (2)..(2)
<223> Xaa can be any naturally occurring amino acid

<400> 3

Cys Xaa Gly Ser Arg Arg Arg Pro Pro Arg Lys Leu
1               5               10


<210> 4
<211> 10
<212> PRT
<213> Artificial

<220>
<223> Heparan sulfate complementary ApoE motif


<220>
<221> misc_feature
<222> (2)..(2)
<223> Xaa can be any naturally occurring amino acid

<400> 4

Cys Xaa Gly Glu Arg Leu Arg Ala Arg Met
1               5               10


<210> 5
<211> 14
<212> PRT
<213> Artificial

<220>
<223> Heparan sulfate complementary AAMP


<220>
<221> misc_feature
<222> (13)..(13)
<223> Xaa can be any naturally occurring amino acid

<400> 5

Arg Arg Leu Arg Arg Met Glu Ser Glu Ser Glu Ser Xaa Cys
1               5               10


<210> 6
<211> 11
<212> PRT
<213> Artificial

<220>
<223> Scramble peptide

<400> 6

Cys Gly Ser Ser Ser Gly Gly Gly Ser Gly Gly
1               5               10